Europäisches Patentamt

European Patent Office

Office européen des brevets

(11) Veröffentlichungsnummer: **0 059 367**
**B1**

(12) **EUROPÄISCHE PATENTSCHRIFT**

(45) Veröffentlichungstag der Patentschrift:
16.05.84

(21) Anmeldenummer: **82101159.0**

(22) Anmeldetag: **17.02.82**

(51) Int. Cl.³: **C 07 C 51/56**, C 07 C 53/12,
C 07 C 51/12, C 07 C 53/08,
C 07 C 51/573, C 07 C 51/48,
C 07 C 67/58, C 07 C 69/14,
C 07 C 45/80, C 07 C 47/06

(54) **Verfahren zur Abtrennung organischer Jod-Verbindungen von Carbonylierungsprodukten des Methanols, Methylacetates und Dimethylethers.**

(30) Priorität: **28.02.81 DE 3107731**

(43) Veröffentlichungstag der Anmeldung:
**08.09.82 Patentblatt 82/36**

(45) Bekanntmachung des Hinweises auf die Patenterteilung:
**16.05.84 Patentblatt 84/20**

(84) Benannte Vertragsstaaten:
**BE DE FR GB IT NL**

(56) Entgegenhaltungen:
**DE - A - 1 197 074**
**DE - A - 3 028 934**

(73) Patentinhaber: **BASF Aktiengesellschaft,
Carl-Bosch-Strasse 38, D-6700 Ludwigshafen (DE)**

(72) Erfinder: **Hartmann, Horst, Lindenstrasse 45,
D-6737 Boehl-Iggelheim (DE)**
Erfinder: **Hochstein, Waldheim, Dr., Am Wurmberg 4,
D-6713 Freinsheim (DE)**
Erfinder: **Kaibel, Gerd, Robert-Bosch-Strasse 4,
D-6840 Lampertheim (DE)**
Erfinder: **Mueller, Franz-Josef, Dr.,
Mueller-Thurgau-Weg 1, D-6706 Wachenheim (DE)**

**0 059 367**

## Verfahren zur Abtrennung organischer Jod-Verbindungen von Carbonylierungsprodukten des Methanols, Methylacetates und Dimethylethers

Die vorliegende Erfindung betrifft ein neues Verfahren zur Abtrennung organischer Jod-Verbindungen, wie vor allem Methyljodid, von Carbonylierungsprodukten des Methanols, des Methylacetates und des Dimethylethers oder von Gemischen solcher Carbonylierungsprodukte.

Es ist allgemein bekannt, daß Methanol, Methylacetat und Dimethylether mit Kohlenmonoxid oder mit Kohlenmonoxid und Wasserstoff in Gegenwart von carbonylbildenden Metallen, hauptsächlich solchen der VIII. Gruppe des Periodensystems, auf vielfältige Weise zu verschiedenen Produkten reagieren können.

Diese Reaktionen werden üblicherweise unter dem Oberbegriff der Carbonylierung zusammengefaßt. Im einzelnen handelt es sich hierbei u. a. um die

— Carbonylierung (im engeren Sinne) von Methanol oder Dimethylether mit u. a. Co-, Ni- oder Rh-Katalysatoren zu Gemischen, die im wesentlichen aus Methanol, Essigsäure und Methylacetat bestehen, die
— Carbonylierung (im engeren Sinne) von Methylacetat mit u. a. Co-, Ni- oder Rh-Katalysatoren zu Gemischen, die im wesentlichen aus Methylacetat und Acetanhydrid bestehen, die
— Homologisierung von Methanol und Dimethylether mit $CO/H_2$ in Gegenwart von u. a. Co-, Ni- oder Rh-Katalysatoren, die je nach den Reaktionsbedingungen zu Gemischen führt, welche neben Methanol im wesentlichen Acetaldehyd, Ethanol, Methylacetat, Acetaldehyddimethylacetal und Wasser enthalten, und um die
— Homologisierung von Methylacetat mit $CO/H_2$ in Gegenwart von u. a. Co-, Ni- oder Rh-Katalysatoren, bei der man je nach den Reaktionsbedingungen Gemische erhält, die im wesentlichen aus Ethylidendiacetat, Vinylacetat, Methylacetat, Ethylacetat, Wasser und Methanol bestehen.

Für alle diese Verfahren sind eine Vielzahl von Varianten bekannt oder denkbar, so daß man entsprechend viele Reaktionsgemische unterschiedlicher qualitativer und quantitativer Zusammensetzung erhält. Den meisten Varianten dieser Carbonylierungsreaktionen ist jedoch gemeinsam, daß sie in Gegenwart von Jodiden vorgenommen werden, so daß die Reaktionsgemische stets merkliche Mengen an jodorganischen Verbindungen wie vor allem Methyljodid enthalten.

Diese jodorganischen Verbindungen lassen sich von den übrigen organischen Komponenten der Carbonylierungsgemische nur äußerst schwer abtrennen.

Die Fraktionierung, wenn wegen vielfältiger Azeotropbildung überhaupt möglich, erfordert einen unwirtschaftlich hohen Trennaufwand, und chemische Methoden wie Oxidation, Reduktion oder Alkalibehandlung kommen wegen der Empfindlichkeit mancher Carbonylierungsprodukte nicht in Betracht, ganz abgesehen davon, daß sie verfahrenstechnisch umständlich sind und meistens nur ein schwieriges Trennproblem durch im Prinzip weniger schwierige ersetzen.

Beispielsweise entfernt man das Methyljodid von Carbonylierungsprodukten des Methylacetats nach der Lehre der DE-OS 29 40 751 durch Umsetzung mit Alkalimetallacetaten, wobei man Alkalimetalljodide erhält. Da diese Umsetzung jedoch nur bei erhöhter Temperatur stattfindet, muß man sie unter Druck vornehmen, und danach müssen die Jodsalze abgetrennt werden. Diese Maßnahmen sowie auch die anschließende Rückführung der Jodide in den Verfahrenskreislauf sind jedoch verfahrenstechnisch aufwendig und unbefriedigend und lassen sich vor allem nicht harmonisch in einen kontinuierlichen Ablauf einfügen. Ist schließlich Wasser als Komponente des Reaktionsgemisches zugegen, gelingt diese Methode der Jodentfernung praktisch überhaupt nicht mehr.

Nach dem Verfahren der DE-A-3 045 105 trennt man Acetaldehyd von jodorganischen Verbindungen wie vor allem Methyljodid ab, indem man den Acetaldehyd zusammen mit einem Kohlenwasserstoff, der unter Normaldruck bei 25—55°C siedet, azeotrop abdestilliert. Die jodorganischen Verbindungen verbleiben in diesem Fall im Destillationsrückstand, und der Acetataldehyd kann wie üblich aus dem Azeotrop gewonnen werden, indem man ihn mit Wasser extrahiert und aus der Extraktphase destillativ isoliert.

Von acetaldehydfreien Carbonylierungsgemischen, z. B. solchen, die nach dem vorgenannten Verfahren vom Acetataldehyd befreit worden sind, können die jodorganischen Verbindungen nach der DE-A-3 045 081 abgetrennt werden, indem man sie mit Hilfe der gleichen Kohlenwasserstoffe durch Azeotropdestillation entfernt.

Der Erfindung lag nun die Aufgabe zugrunde, Methyljodid sowie sonstige organische Jod-Verbindungen aus Reaktionsprodukten der Carbonylierung von Methanol, Methylacetat und Dimethylether oder von Gemischen solcher Carbonylierungsprodukte auf eine weitere einfache und wirtschaftliche Weise zu entfernen.

Demgemäß wurde gefunden, daß man Methyljodid sowie sonstige organische Jod-Verbindungen auf elegante Weise von Reaktionsprodukten der Carbonylierung von Methanol, Methylacetat und Dimethylether oder von Gemischen solcher Carbonylierungsprodukte abtrennen kann, wenn man die Jod-Verbindungen durch Flüssigphasen-Extraktion mit einem nicht-aromatischen Kohlenwasserstoff entfernt.

Überraschenderweise ist das erfindungsgemäße Verfahren nicht nur auf bestimmte Carbonylierungsprodukte des Methanols, Methylacetats und Dimethylethers anwendbar, sondern auch auf beliebige Gemische, die bei diesen Reaktionen anfallen. Die weitere Aufarbeitung solcher Gemische ist somit nicht mehr mit dem Problem der Jodentfernung belastet und kann daher auf beliebige Weise vorgenommen werden.

Für das erfindungsgemäße Extraktionsverfahren verwendet man pro Gramm der Jodverbindungen — hauptsächlich handelt es sich um Methyljodid — etwa 100—3000 g des Kohlenwasserstoffs. Als nicht-aromatische Kohlenwasserstoffe eignen sich alle diejenigen, die unter den Extraktionsbedingungen flüssig sind. In Betracht kommen daher geradkettige oder verzweigte $C_4$—$C_{14}$-Alkane, mono-, bi- und tricyclische Cycloalkane mit 5 oder 6 C-Atomen pro Ring, sowie auch Gemische dieser Kohlenwasserstoffe. Beispiele für derartige Kohlenwasserstoffe sind Isopentan, 2,2,4-Trimethylpentan, n-Octan, n-Nonan, n-Decan, Cyclopentan, Cyclohexan und Decahydronaphthalin.

Allgemein haben die definitionsgemäßen Kohlenwasserstoffe ein ausgeprägt selektives Lösevermögen für die jodorganischen Verbindungen, besonders für das bei den hier in Rede stehenden Carbonylierungsgemischen vornehmlich auftretende Methyljodid. Die übrigen Komponenten gelangen dagegen umso weniger in die Extraktphase je polarer sie sind. So werden Wasser, Essigsäure, Ethanol, Acetaldehyd und Methanol praktisch nicht extrahiert, wogegen geringe Mengen von Dimethylether, Methylacetat und Acetaldehyddimethylacetal auch in die Extraktphase gelangen können. Durch Wasserzusatz — etwa 20—200 Gew.-% des zu extrahierenden Produktes bzw. Produktgemisches — kann der Wirkungsgrad der Extraktion häufig verbessert werden.

Vorzugsweise nimmt man die Extraktion bei Normaldruck zwischen 0 und 30° C vor. Leicht erhöhter Druck — etwa bis zu 4 bar — kann erforderlich sein, wenn man einen $C_4$-Kohlenwasserstoff wie Butan oder Isobutan verwendet. Weiterhin erhöht sich der Druck — etwa bis zu 10 bar — wenn man bei höheren Temperaturen — etwa bis zu 70—80° C — arbeitet.

Man kann die Extraktion diskontinuierlich oder vorzugsweise kontinuierlich in allen hierfür üblichen Apparaturen vornehmen, z. B. mittels Mixer-Settler-Apparaturen oder Extraktionskolonnen beliebiger Bauart, wobei der Kohlenwasserstoff von unten nach oben im Gegenstrom zu dem Carbonylierungsgemisch geführt wird. Führt ein einziger Extraktionsvorgang nicht zu der gewünschten Abreicherung der jodorganischen Verbindungen, so kann man wie üblich mehrere — etwa bis zu 10 — Extraktionsstufen in Serie schalten.

Zur Aufarbeitung der Extraktphase destilliert man die mengenmäßig geringen Jodverbindungen von dem Extraktionsmittel ab und führt sie zweckmäßigerweise in die Carbonylierungsstufe zurück. Hierbei in das Destillat gelangendes Extraktionsmittel und eventuell mitextrahierte Carbonylierungsprodukte können ebenfalls in die Carbonylierungsstufe zurückgeführt werden, da sie dort nicht stören.

Ferner kann die Jodverbindung auf beliebige chemische Weise, z. B. durch Behandlung mit Alkalimetallen oder Alkalilaugen und anschließende Abtrennung der Jodide von den inerten Kohlenwasserstoffen, entfernt werden.

Die Entfernung von Extraktionsmittelresten aus der Raffinatphase gestaltet sich einfach, sofern man ein Extraktionsmittel wählt, welches, wie stets möglich ist, eine genügende Siedepunktsdifferenz gegenüber den Komponenten der Raffinatphase oder deren Folgeprodukten aufweist, so daß leicht eine destillative Trennung vorgenommen werden kann.

Das erfindungsgemäße Verfahren ist prinzipiell unabhängig davon, unter welchen Bedingungen und mit welcher Zielsetzung die Carbonylierung vorgenommen wurde, sofern überhaupt Jod-Verbindungen als Aktivatoren im System der Carbonylierungskatalysatoren mitverwendet wurden, wie es für die Mehrzahl der technisch ausgeübten Synthesen dieser Art zutrifft. Es erübrigt sich daher, die verschiedenartigen Reaktionsbedingungen für die eingangs erwähnten Varianten der Carbonylierungsreaktionen im einzelnen aufzuführen.

Das erfindungsgemäße Verfahren ermöglicht es auf denkbar einfache Weise, den Jodgehalt der Carbonylierungsprodukte bzw. von deren Gemischen von etwa 1000—10 000 ppm auf 0,01—20 ppm zu senken.

Beispiele 1—12

Je m(g) eines Carbonylierungsproduktes bzw. eines Gemisches solcher Produkte mit dem Methyljodidgehalt p (ppm) wurde mit je 100 g eines Kohlenwasserstoffs als Extraktionsmittel bei T °C n-mal ausgeschüttelt. Danach wurden die durch die Extraktion entstandenen Verluste der Produkte sowie der Methyljodidgehalt nach der n-ten Extraktionsstufe ermittelt. Der Methyljodidgehalt wurde hierbei gaschromatographisch bestimmt.

# 0 059 367

Die Einzelheiten dieser Versuche sowie deren Ergebnisse sind der Tabelle zu entnehmen.

| Bsp. Nr. | Zu extrahierendes Produkt(gemisch) P Art | Menge m (g) | CH$_3$J-Gehalt p (ppm) | Extraktions-mittel | Extrak-tions-stufen n | Tem-pe-ratur °C | Raffinatphase Verlust an P (g) | CH$_3$J-Gehalt (ppm) |
|---|---|---|---|---|---|---|---|---|
| 1 | Essigsäure | 100 | 5000 | Decahydro-naphthalin | 5 | 25 | 3 | 86 |
| 2 | Essigsäure Wasser | 75 50 | 2900 | Decahydro-naphthalin | 5 | 25 | 0,3 | 0,2 |
| 3 | Essigsäure Methylacetat | 67 33 | 4700 | Decahydro-naphthalin | 4 | 25 | 3 3 | 210 |
| 4 | Essigsäure Methylacetat Wasser | 50 50 50 | 3400 | Decahydro-naphthalin | 5 | 25 | 0,2 2 | 0,1 |
| 5 | Acetanhydrid Methylacetat | 50 50 | 3900 | Decahydro-naphthalin | 5 | 25 | 1 2 | 15 |
| 6 | Acetanhydrid Methylacetat Ethylidendiacetat | 33 33 33 | 6500 | Decahydro-naphthalin | 5 | 25 | 0,2 2 1,5 | 63 |
| 7 | Acetaldehyd Wasser | 100 100 | 150 | Isopentan | 3 | 10 | 2 | 0,2 |
| 8 | Acetaldehyd Wasser | 100 100 | 280 | 1,2,4-Tri-methylpentan | 3 | 18 | 2 | 0,6 |
| 9 | Acetaldehyd Wasser | 100 100 | 280 | n-Nonan | 3 | 18 | 1,5 | 0,8 |
| 10 | Acetaldehyd Wasser | 100 100 | 280 | n-Octan | 3 | 18 | 1,5 | 0,8 |
| 11 | Acetaldehyd Wasser | 100 100 | 280 | Decahydro-naphthalin | 3 | 18 | 1 | 0,6 |
| 12 | Acetaldehyd Wasser | 100 100 | 280 | n-Decan | 3 | 18 | 1,5 | 0,5 |

## Beispiel 13

In einer kontinuierlich betriebenen 3stufigen Mixer-Settler-Apparatur wurde bei 20°C stündlich 500 g eines Carbonylierungsgemisches im Gegenstrom mit 250 g n-Decan extrahiert.

Das Carbonylierungsgemisch wurde durch Carbonylierung von Methanol mit Synthesegas (CO : H$_2$ = 1,3 : 1) in Gegenwart von Cobaltcarbonyl als Katalysator und Natriumjodid als Aktivator bei 185°C und 300 bar hergestellt. Nach der oxidativen Entcobaltung wurden die flüssigen Bestandteile mit Waser verdünnt, wonach das Gemisch folgende Zusammensetzung hatte:

50% Wasser (Gew.-% wie im folgenden), 5% Acetaldehyd, 9% Acetaldehyddimethylacetal, 8% Methylacetat, 26% Methanol und 2% Ethanol. Dieses Gemisch enthielt 120 ppm Methyljodid.

Nach der Extraktion enthielt die Raffinatphase nur noch 0,4 ppm Methyljodid. Die durch die Extraktion entstandenen Verluste an Carbonylierungsprodukten betrug rd. 1—2% Methylacetat und rd. 2—3% Acetaldehyddimethylacetal.

4

**0 059 367**

**Patentanspruch**

Verfahren zur Abtrennung organischer Jod-Verbindungen von Carbonylierungsprodukten des Methanols, Methylacetates und Dimethylethers oder von Gemischen solcher Carbonylierungsprodukte, dadurch gekennzeichnet, daß man die Jod-Verbindungen durch Flüssigphasen-Extraktion mit einem nicht-aromatischen Kohlenwasserstoff entfernt.

**Claim**

A process for separating organic iodine compounds from carbonylation products of methanol, methyl acetate and dimethyl ether or from mixtures of such carbonylation products, wherein the iodine compounds are removed by liquid phase extraction with a non-aromatic hydrocarbon.

**Revendication**

Procédé pour la séparation de composés organiques de l'iode d'avec des produits de carbonylation du méthanol, de l'acétate de méthyle et de l'éther diméthylique ou de mélanges de ces produits de carbonylation, caractérisé en ce qu'on élimine les composés de l'iode par extraction en phase liquide avec un hydrocarbure avec non aromatique.